# EUROPEAN PATENT APPLICATION

(11) **EP 1 849 440 A1**
(43) Date of publication of application: **31.10.2007**
(21) Application number: 06290707.6
(22) Date of filing: 28.04.2006
(51) Int. Cl.: A61F 2/90, A61F 2/84

(54) **Vascular stents with varying diameter**

(71) Applicant: Boudjemline, Younes, 94000 Creteil (FR)
(72) Inventor: Boudjemline, Younes, 94000 Creteil (FR)
(74) Representative: Crump, Julian Richard John

(57) **Abstract**

Embodiments of the present invention are directed to medical implants/stents and system. Some embodiments of the invention include a self-expanding stent having a tabular first wall structure of a first diameter, where each end portion of the first wall structure is formed into a second wall structure of a second diameter larger than the first diameter. A membrane covers at least the first wall structure and the stent includes an interlaced, helicoidally wound wire forming a mesh-like structure.

## Description

### Field of the Invention

Embodiments of the present invention are directed generally to stent devices, and more particularly, to stents for vascular applications, including diametrical reduction and valve replacement in vascular applications. Some of the embodiments of the present invention are also directed to applications and methods of use for such stents, as well as methods of manufacture for such stents.

### Background of the invention

It is well-known that a section of the lumen of a corporeal duct may be restored by means of a tubular extension. Such an extension, which may be referred to as a "stent", is deformable between a contracted state and a deployed state. In a contracted state, the stent is capable of introduction and movement along a corporeal duct up to the site to be treated, while the deployed state enables the stent to rest against the wall of the conduit at the site to be treated and restores the section of the conduit. Such a stent may also be used for implanting a prosthetic system in a corporeal duct, for instance a cardiac valve, or to isolate an arterial hernia.

It is also well-known to plug a hole in a corporeal wall by means of a two-collar implant, currently denominated as "plug", each of these collars resting against one of the faces of the wall to be treated.

There exist numerous models of stents or of plugs, notably stents formed by laser-cutting a thin sheet of appropriate metal material or formed by braiding several metal wires, notably made of memory-shape alloy. The shortcoming of these stents and plugs lies in their being relatively difficult to produce.

One of the shortcoming of known stents is their lack of adaptability with regard to variations in diameter of vessels in which they are used. Thus, stents of different diameters must be produced for treating different corporeal ducts having different diameters. Moreover, current stents include ends which are relatively aggressive which may have significant damaging consequences.

The document EP 0 857 471 describes several structures of stents, where two with a "trellis mesh" are difficult to produce and exhibit no adaptability of diameter or of shape.
This document also describes a stent formed by a single wire whereof each strand runs helicoidally from one end to the other of the stent and is braided to the others strands. At the ends of the stent, each strand connects to the following strand by an elbow. Though this design addresses some concerns of prior art stents, the embodiments disclosed in the document do not address concerns related to the adaptability of the diameter, the shape of the stent and the character of the aggressiveness of the ends of stents.

The document US 20021169498 describes a stent with a "trellis mesh" structure, considered as difficult to produce and exhibiting no adaptability of diameter or of shape.

### SUMMARY OF THE INVENTION

Accordingly, embodiments of the present invention address the above noted concerns of prior art implants (e.g., stents). To that end, some of the embodiments of the present invention remedy the shortcomings of the prior art stents and provide stents which can be adapted to a plurality of different diameter vessels and openings. Embodiments of the present invention may be implanted into a patient via any one of transcatheter, percutaneous, transventricular, transatrial and trans-vacular/mini-invasive insertion.

To that end, some embodiments of the present invention provide a medical implant such as a stent which reduces the diameter of a vascular structure. Such a reduced diameter may be used for the placement of a valve either directly, or upon placement of a second stent in the reduced diameter. Such embodiments may also include a covering (e.g., PTFE membrane) to avoid blood going in between the wires of the stent. Such embodiments allow a gradient between the proximal and distal portions of the vessel area to which the stent is positioned. In addition, such stents may be used with a second stent in the restricted diameter (for example), for deploying a valve or other device.

Some embodiments of the present invention provide a method of production of a medical implant with mesh-like structure, notably a "stent" or a "plug", relatively easy to implement and enabling the realization of implants which are perfectly functional.

With regard to the method of manufacturing stents according to some embodiments of the present invention, one method may include forming a stent structure by running a strand of wire helicoidally from one end to the other of the structure and interlacing the strand with other strands previously arranged. Such a method may include forming a loop between each strand at each end of the structure and setting the free ends of the first and of the last strand significantly back from the ends of the structure.

Another embodiment of the invention is a method which may include: using a single wire to form a tubular mesh-like structure, forming a first strand wherein the free end of the first strand is set back from a first location corresponding to a first end of the structure. The method also includes running the first strand along a helicoid path up to a second location corresponding to a second end of the structure, the first strand forming a loop at this second location, thus singling out a second strand. The method may further include one or more (and preferably all) of the following additional steps:
- running the second strand along a helicoid path up to the first location, by interlacing the second strand with the first strand when it meets the latter, the second strand forming a loop at the first location singling out a following strand;
- running the following strand along a helicoid path up to the opposite location, by interlacing the following strand with the front strand(s) on its way, the following strand forming a loop at the opposite location singling out a second following strand;
- repeating the operations from the above step a plurality of times necessary to form a mesh-like tubular structure and loops on the whole circumference of the locations, up to singling out a last strand; and
- interlacing the last strand with the previous strand(s) on its way and interrupting this last strand so that its free end is set significantly back from the opposite location.

Producing a structure from a single wire, combined to the arrangement of the loops between each strand of wire and to the setting of the free ends of the first and of the last strand significantly back from the ends of the structure, enables the strands to be slid against one another in some embodiments of the invention. This sliding motion may be made possible by, for example, clamping or expanding loops, according to the diameter or the shape given to the structure. The latter is preferably deformable in its diameter as well as in its shape, and remains non-aggressive to the walls of a corporeal duct regardless of the diameter and/or the shape given thereto.

The absence of welded spots between the strands and the deformability of the loops in some embodiments of the present invention also has as an advantage of enabling significant variation of the angles formed by the strands therebetween. The multiples sides of these strands enable wider variability of the different diameters which the structure may exhibit, and hence the production of a stent having wider range of variations in diameter. Accordingly, this allows such stents to be used for treating a wider range of diameters of corporeal ducts.

The loops formed by the wire at the ends of said structure partake of these wider possibilities of deformation and are moreover non-aggressive for the wall of the corporeal duct treated. The setting of the free ends of the first and of the last strand back from the ends of the stent enable many adaptations of the diameter and/or of the shape of the stent without risking that these ends protrude beyond the ends of the stent and should not form sharp excrescences for the corporeal duct to be treated.

The formed structure may also be used as a blank for the production of a stent or of a "plug" of specific shapes. The method may then comprise: deformation of the tubular structure to form a specific shape of stent or of the "plug" to produce (and provide stabilization) the tubular structure in the new shape.

Preferably, interlacing a strand with other encountered strands is performed as a braiding process, i.e. this strand runs alternately on a strand on its way then under the following strand, and so on. This braiding allows the structure to be used as a stent or to serve as a blank for the production of other implants (e.g., plugs). The braiding also enables a reliable stop of the first and of the last strands formed by the wire.

The wire used may be a shape memory alloy, in particular a nickel-titanium alloy, known under the designation "NITINOL", having a diameter ranging from, for example, 0.15 to 0.5 mm. The diameter of the structures which may be produced by the method according to the invention very widely, and may range from 5 to 100 mm (for example). The method may include the step of placing on the structure a means for longitudinal shortening of the structure - that is, the ability to switch from an elongated state to a shortened state. Longitudinal shortening may also enable the deployment of the structure or facilitate the deployment of the structure. The means for accomplishing longitudinal shortening may include an elastic means - for instance, a band made of elastic material, notably of silicon. The elastic material may be a shape memory alloy enabling the switch from an elongated state to a shortened state by changing the temperature of the body.

At least one radio-opaque wire or marker may be added to the structure of a stent according to any of the embodiments of the invention, and particularly to the wire-based structures. Such radio-opaque markers may be comprised of: any metallic thread having sufficient cross-sectional area to perform the intended function (e.g., between about 0.2 and 1.0 mm). The radio-opaque marker increases the radio-opacity of a stent in one or more areas to help orientate the device. For example, in a valved stent/implant, the radio-opaque marker(s) may be placed in a strategic area of the stent (e.g., the front of one or more commissures) of the stent. Such placement of the marker allows for an ideal orientation of the valved stent since the maker can be related to the commissure of the patient valve being replaced. Specifically, a surgeon need rotate the delivery system of the stent to align the marker with the area of interest. The commissure of the patient may be native or bioprosthetic if the patient has already undergone a replacement of a cardiac valve.

The longitudinal shortening means may be engaged through two loops formed at the ends of the structure. The method may include covering the structure with a watertight flexible wall, using, for example, a Teflon sheet, which may be sewed to the structure. Because of the watertight aspect, the structure may be used to isolate an arterial hernia when in place.

Another embodiment of the present invention is directed to a medical implant which may include a stent having a tubular first wall structure of a first diameter, where at least one end portion of the first wall structure is formed into a second diameter.

Another embodiment of the present invention is directed to a medical implant which may include a self-expanding stent having a tubular first wall structure of a first diameter, where each end portion of the first wall structure is formed into a second wall structure of a second diameter larger than the first diameter. The implant may also include a membrane covering for covering at least the first wall structure, and the stent may be formed of interlaced, helicoidally wound wire forming a mesh-like structure.

Another embodiment of the present invention is directed to an implant system comprising a first self-expanding stent which may include a tubular first wall structure of a first diameter, where at least one end portion of the first wall structure is formed into a second diameter. The system may also include a second balloon expandable stent comprising a tubular structure, where the balloon expandable stent may be deployed within a portion first wall structure.

Another embodiment of the present invention is directed to a method for implanting a medical device and may include providing a self-expanding stent capable upon expansion of forming a tubular first wall structure of a first diameter, wherein each end portion of the first wall structure form into a second wall structure of a second diameter larger than the first diameter, loading the self-expanding stent into a delivery system, the stent being enclosed by a sheath, inserting the delivery system over a guide-wire, a portion of the guide-wire lying adjacent an area of interest for implanting the stent, advancing the stent to the area of interest, deploying a distal end of the stent out from the sheath, pushing the delivery system in distal end direction such that distal end of the stent is turned backward toward the proximal end of the stent, deploying the tubular first wall structure of the stent, and deploying the proximal end of the stent.

Another embodiment of the present invention is directed to a method for implanting a medical device. The method may include deploying a first stent within a designated area of a patient, the first stent comprising a tubular first wall structure of a first diameter, where at least one end portion of the first wall structure is formed into a second diameter. The method may also include deploying a second stent comprising a second tubular structure within the first wall structure of the first stent.

Another embodiment of the invention is directed to a method for implanting a medical device may include deploying a first self-expanding stent within a designated area of a patient, where the first stent comprising a tubular first wall structure of a first diameter. At least one end portion of the wall may be formed into a second diameter. The method may also include calibrating the wall structure of the first diameter to a predetermined diameter.

Another embodiment of the present invention is directed to a method for treating a staged right ventricular outflow tract stenosis of a patient. The method may include deploying a first stent proximate a ventricular setpal defect of a patient, where the first stent comprises a tubular first wall structure of a first diameter, the at least one end portion of the first wall structure may be formed into a second diameter and a conical portion, at least the conical portion includes a membrane covering, and the defect may be sealed by the membrane/conical portion.

Another embodiment of the invention is directed to a method for closing a cardiac defect in a patient and may include providing a stent comprising a self-expanding medical implant having a tubular structure of a first diameter and at least one end portion formed into a disk having a second diameter, where the stent includes a membrane covering at least the disk. The method may also include deploying the stent within the cardiac defect.

Another embodiment of the invention is directed to a self-expanding valve replacement implant for a patient and may include a first tubular wall structure of a first diameter and a Valsalva portion corresponding in shape to the Valsalva portion of the valve area of the valve being replaced in a patient.

Another embodiment of the invention is directed to a medical implant which may include a first stent having a first tubular wall structure of a first diameter and a second separate stent comprising a second tubular wall structure of a second diameter. The at least one of the first stent and the second stent may include a plurality of prolongations which connect the first stent and the second stent in spaced apart arrangement.

Another embodiment of the present invention is directed to a method of restricting a diameter of a vessel. The method may include providing a self-expanding stent having a tubular first wall structure of a first diameter, where each end portion of the first wall structure is formed into a second wall structure of a second diameter larger than the first diameter and a membrane covers at least the first wall structure. The method may also include deploying the stent within a vessel.

In yet another embodiment of the present invention, a system for implanting a medical device is provided, and may include a self-expanding stent capable upon expansion of forming a tubular first wall structure of a first diameter, where each end portion of the first wall structure form into a second wall structure of a second diameter larger than the first diameter, a delivery system having a sheath capable of loading the self-expanding stent, and a guide-wire upon which the delivery system is guided to an area of interest in a patient for implanting the stent. Upon the delivery system being advanced to the area of interest, the distal end of the stent is deployed from the sheath, the delivery system is pushed in the distal end direction such that distal end of the stent is turned backward toward the proximal end of the stent.

The invention will be better understood, and other characteristics and advantages thereof will appear, with reference to the appended schematic drawing, representing, for non limiting exemplification purposes, several structures of implant obtained by the method concerned.

### BRIEF DESCRIPTION OF THE DRAWINGS

Figs. 1-4 are perspective views of a device used for implementing this method, showing respectively four successive steps contained in this method.

Fig. 5 is a perspective view of the mesh-like tubular structure obtained. For clarity of the drawing, this structure is fictitiously represented as opaque, the portions at the foreground masking the portions at the background.

Fig. 6 is a view of said structure similar to Fig. 5, below another angle, the structure being fitted with an elastic wristband forming a longitudinal shortening means.

Fig. 7 is a perspective view of another device used for implementing this method.

Fig. 8 is a perspective view of this device with placement of a mesh- like tubular structure thereon.

Fig. 9 is a view of this mesh-like tubular structure, after retraction outside the device ; here also, this structure is fictitiously represented as opaque.

Figs. 10-12 are face, side and sectional views, respectively, after placing on a corporeal wall, of an implant obtained from the mesh-like tubular 30 structure shown on Fig. 9, this implant being intended for blanking a hole existing in a corporeal wall.

Figs. 13-14 are side and sectional views, respectively, after placing on a corporeal wall, of another implant obtained from of the mesh-like tubular structure shown on Fig. 9, this implant being also intended for blanking a hole existing in a corporeal wall or replacing a cardiac valve.

Figs. 15-16 are side views of both examples of mesh-like tubular structures which may be obtained by the method according to the invention.

Figs. 17A-C illustrate lateral, front-uncovered and front covered views, respectively, of a stent for a cardiac closure application according to an embodiment of the present invention.

Figs. 18A-D illustrate lateral and front views, both uncovered and covered, of a stent for diametrical reduction of an opening/vessel, according to an embodiment of the present invention.

Fig. 19 illustrates an uncovered, lateral view of a stent containing a valve according to an embodiment of the present invention.

Figs. 20A-G illustrate cross-sectional shapes and positions of end portions of stents according to some embodiments of the present invention.

Fig. 21A illustrates a lateral, uncovered view of a stent according to an embodiment of the present invention.

Fig. 21B illustrates a lateral, uncovered view of a stent according to an embodiment of the present invention.

Fig. 21C illustrates a lateral, uncovered view of a stent according to an embodiment of the present invention, having a membrane positioned between two end portions.

Fig. 22 illustrates a lateral, uncovered view of a stent according to an embodiment of the present invention.

Fig. 23A illustrates a delivery means including a stent according to one embodiment of the present invention in an undeployed state.

Fig. 23B illustrates the delivery means of Fig. 23A, having a distal end of the stent deployed.

Fig. 23C illustrates the delivery means of Figs. 23A, having the stent fully deployed.

Figs. 24A-D are x-ray images of the deployment in a patient of a first stent system according to one embodiment of the present invention.

Figs. 25A-D are x-ray images of the deployment in a patient of a second stent system according to one embodiment of the present invention.

Fig. 26A is a cross-sectional illustration of a ventricular setpal defect.

Fig. 26B is a lateral view of a stent for treating a ventricular setpal defect (VSD) according to an embodiment of the present invention.

Fig. 26C is another lateral view of the stent of Fig. 26B, over-laid on a cross-sectional image of a VSD.

Fig. 27 is a lateral uncovered view of a stent according to an embodiment of the present invention.

Fig. 28A is a lateral uncovered view of a stent according to an embodiment of the present invention.

Fig. 28B is a top uncovered view of the stent of Fig. 28A, with a closed valve.

Fig. 28C is a lateral uncovered view of the stent of Fig. 28A, with a valve.

Fig. 29A is a lateral, uncovered view of a stent according to an embodiment of the present invention.

Fig. 29B is a lateral, uncovered view of the stent of Fig. 29A, including a valve.

Fig. 29C is a top, uncovered view of the stent of Fig. 29A, with a valve in an open position.

Fig. 29D is a top, uncovered view of the stent of Fig. 29A, with a valve in a closed position.

Fig. 30A is a lateral, uncovered view of a first stent according to an embodiment of a stent system for the present invention.

Fig. 30B is a lateral, uncovered view of the first stent and the second stent of the stent system of Fig. 30A, where the second stent includes a valve.

Fig. 30C is a top, uncovered view of the first and second stent of Fig. 30B, with the valve in the open position.

Fig. 30D is a top, uncovered view of the first and second stent of Fig. 30B, with the valve in the closed position.

Figs. 31A-C are top uncovered, lateral uncovered and top covered views of a stent according to an embodiment of the present invention.

Figs. 32A-C correspond to Tables 1-3, respectively, illustrating data collected in an experiment using

### DETAILED DESCRIPTION OF THE EMBODMIENTS

This application is a continuation-in-part to U.S. patent application no. 10/514,329, filed July 6, 2005, which is a national stage application of PCT application no. PCT/FR03/03296, filed November 5, 2003, which claims priority to French application no. FR 02 14522, filed November 20, 2002. Each of the foregoing disclosures is herein incorporated by reference.

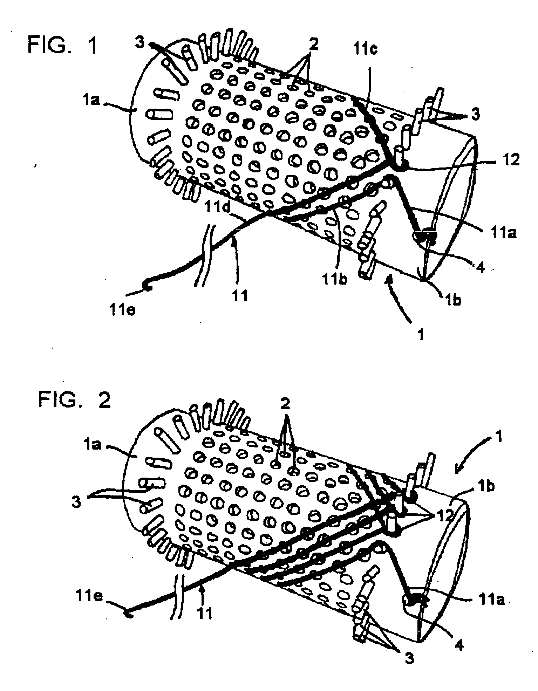

While some of the embodiments of the present invention are described herein as being manufactured according to the structures illustrated in Figs. 1-8, and corresponding methods described herein, some embodiments of the present invention may also be manufactured using laser micromaching/cutting.

For simplification purposes, the portions or element present on the different devices and structures will be designated by the same numeric references and will not be described again. Fig. 1 represents a tubular chuck 1 drilled with holes 2 evenly distributed on its wall, these holes 2 being preferably aligned longitudinally and transversally. On its longitudinal ends 1a, 1b, the chuck 1 may comprise a series of holes evenly distributed on its circumference, receiving frictional fit, and preferably removable cylindrical studs 3.

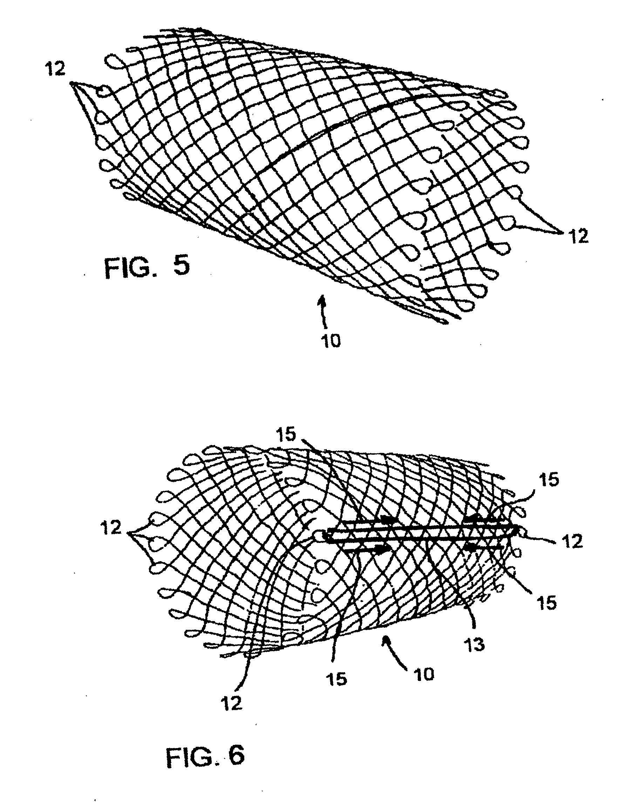

The chuck 1 may also comprise a hole 4 provided slightly recessed from one of its ends 1b. The chuck 1 is intended to be used for producing a mesh-like tubular structure 10 as shown on Figs. 5 and 6, by means of, preferably, a single metal wire 11. This wire 11 is notably made of shape memory alloy known under the designation "NITINOL", or other alloy/material with similar characteristics.

To produce the structure 10, an appropriate length of wire 11 is cut, for instance four meters, and one end 11a of wire is attached to the chuck 1 by engagement in the hole 4 and around the end edge of the chuck 1 then twisting this end 11a around itself.

The wire 11 may then be run around a stud 3 of the end 1b slightly offset angularly, then along the wall of the chuck 1, along a helicoid path running above holes 2 aligned on this path. The first strand 11 b of wire thus formed runs along the wall of the chuck 1 then is engaged around the stud 3 corresponding to the end 1a, by forming a loop around this stud 3, thus singling out a second strand 11c.

As shown on Fig. 1, this second strand 11c is run along the wall of the chuck 1 along a helicoid path until it comes back to a corresponding stud 3 of the end 1b and form a loop 12 around the latter, thus singling out a following strand 11d. In the example represented, the number of holes 2 and of studs 3 is determined so that this second strand 11c comes back to the stud 3 adjoining the stud 3 around which is engaged the previous strand 11b.

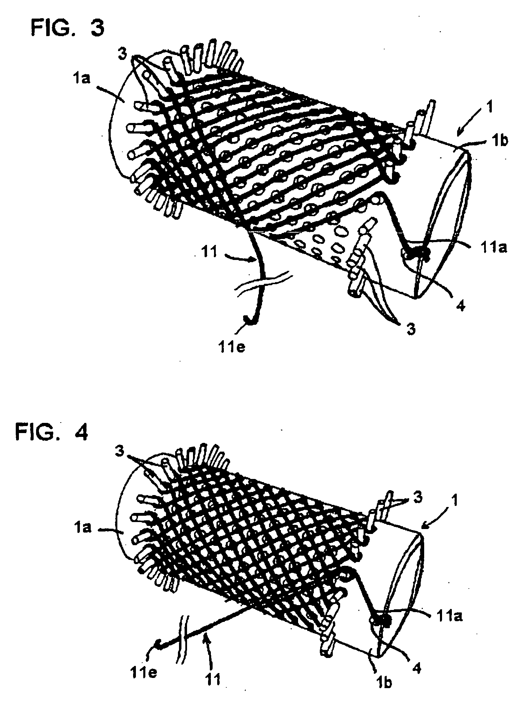

As can be deduced from Figs. 2 and 3, these engagement operations of a strand along the wall of the chuck 1 via a helicoid path, thereby forming a loop 12 around a corresponding stud 3 are repeated as many times as necessary for the formation of the tubular mesh-like structure 10, visible on Fig. 4 whereas it is practically finished.

Each strand is braided with the others strands on its way, i.e. runs alternately over a strand on its way then below the following strand, and so on. This braiding is facilitated by the holes 2 and by the conformation of the free end 11e of the wire 11 into a hook. The last strand is braided with the strands on its way, then the end of this strand is cut to the desired length, so that it is set back from the corresponding end of the chuck 1, i.e. the end 1a in the example represented.

The first strand 11b is then cut to the desired length, so that its end is set back from the end 1b, then the studs 3 are extracted from the holes which receive said studs in order to free the structure 10 and to enable to remove said studs from the chuck 1 by a sliding motion.

According to such embodiments, the structure 10 thus constituted does not comprise any welding spots between the strands of wire 11, nor braids at its ends, but loops 12. The absence of welding spots between the strands and the existence of these loops 12 enable to slide the strands against one another when antagonistic stresses are exerted transversally on the structure 10, and this sliding enables a significant variation of the angles formed by the strands therebetween and hence of the diameter which said structure 10 may acquire.

The latter may be used as such and constitute an extension of corporeal duct currently denominated as "stent". After production as aforementioned, it is exposed in such a case to one or several thermal treatments enabling to stabilize its form and to confer supra-elastic properties thereto.

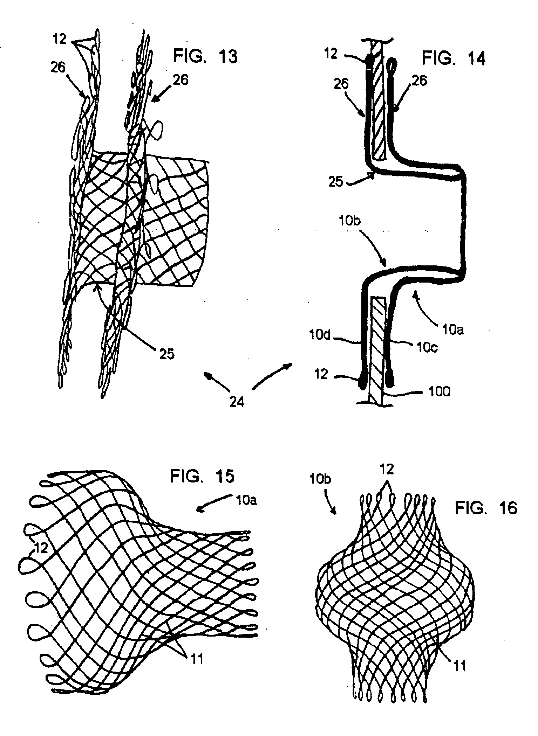

This stent has hence wider possibilities of variations in diameter, which enable it to be used for treating a wider range of diameters of corporeal ducts. The structure 10 may also be deformed to constitute a stent of smaller or of larger diameter, or a stent of particular shape, for instance with a median narrowing. An appropriate contention device, holding the structure 10 in the shape to obtain before thermal treatment, is used in each case, i.e. a contention tube for the production of a stent of smaller diameter, a chuck of diameter larger than the chuck 1 for the production of a stent of larger diameter, or an appropriate shape in the other cases. Figs. 15 and 16 show in this view two examples of mesh-like structures 10A, 10B obtained by braiding on a chuck of appropriate shape or by deformation of the structure 10 then thermal treatment thereof in deformed condition, i.e. a structure 10A whereof one end is flared and a structure 10B whereof the median zone is bulged. The structure 10A may notably serve as a stent for treating a Fallot tetralogy, and the structure 10B may notably serve as an aortic stent for placing an aortic valve, the bulged zone being adaptable to the Valsalva sinus.

Fig. 6 shows a structure 10 obtained as described previously, where a wristband 13 made of silicon is placed thereon, engaged through two loops 12 substantially aligned longitudinally. This wristband 13 is elastic and is stretched when the structure 10 is in a radial contraction condition, taking into account the closing of the angles formed by the strands therebetween during this contraction, and hence the increase in length of the structure 10. When this contraction is released, when placing the implant formed by this structure, the wristband 13 tends to regain its non-stretched shape, as shown by the arrows 15. This wristband 13 provides consequently, and readily, a longitudinal shortening means of said structure 10, which enables or promotes the deployment of this structure 10.

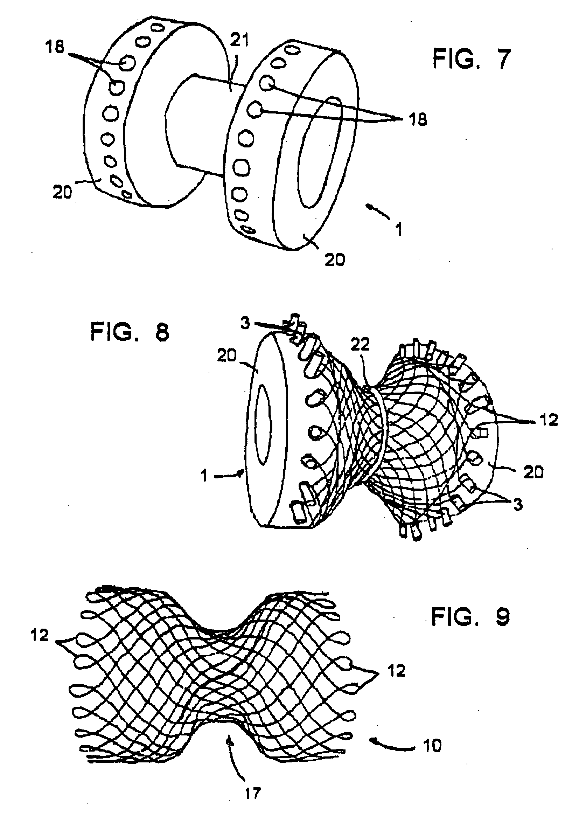

Figs. 7 to 9 show a chuck 1 designed to enable the production of a structure of stent 10 shown on FIG. 9, comprising a central narrowing 17. The chuck 1 comprises in this case two portions 20 of longitudinal ends of larger diameter and a median portion 21 of smaller diameter. The portions 20 comprise the holes 18 receiving the studs 3.

One of the portions 20 is preferably dismountable with respect to the portion 21, to enable retraction of the structure 10 obtained outside the chuck 1. A structure 10 as shown on Fig. 5 is placed on this chuck 1, the length of the latter being such that the strands extend loosely between the studs 3 to enable the arrangement of said narrowing 17. The loops 12 enable perfect maintenance of the structure 10 on the chuck 1 by means of the studs 3.

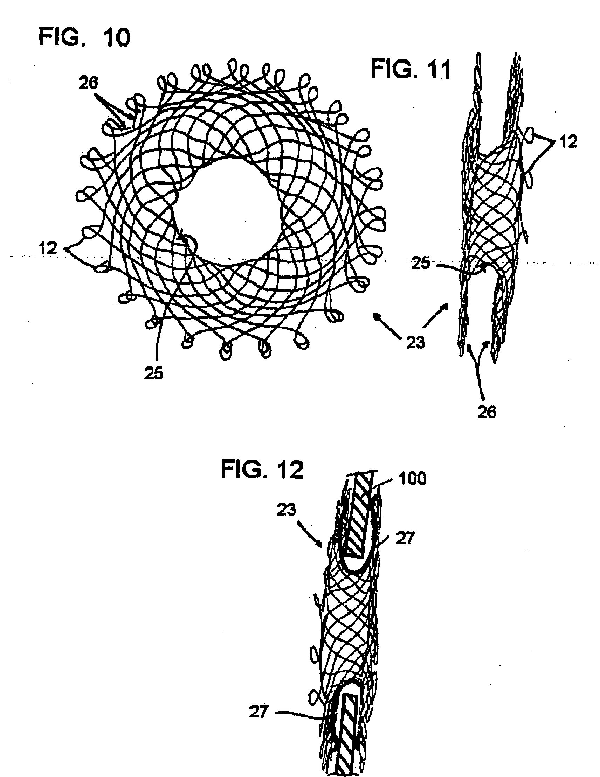

One or several contention wires 22 is then used to form the narrowed median portion 17 of the structure 10, as shown on Fig. 8, to shape the stent adequately and to keep its shape during the single or various subsequent thermal treatments. The stent thus obtained is notably intended to place a prosthetic valve in a corporeal duct. It is preferably covered with a watertight sheet, notably made of PTFE/Teflon®. The structure 10 with narrow portion 17 shown on FIG. 9 may also serve as a blank for the production of implants 23, 24 as shown on Figs. 10 to 14.

The implant 23 is of the type currently designated as a "plug", liable to plug a hole in a corporeal wall 100, for example, notably an interventricular hole in a heart. The implant includes a median portion 25 intended to be engaged in said hole, one or two collars 26 adjoining this central portion 25, liable to rest against said wall 100, on both sides thereof, and a material sheet blanking the opening formed by the median portion 25, notably a Teflon sheet.

In the case of this implant 23, shown on Figs. 10 to 12, both end portions of the structure 10 may be folded radially towards the outside of this structure, to form both collars 26. This deformation is made possible by the deformation properties of the structure 10 detailed previously. The structure 10, thus deformed, is placed in a contention temps, holding it in this position in order to carry out the single or various thermal treatments aforementioned.

Fig. 12 shows that the implant 23 may receive one or several elastic clips 27 maintaining both collars 26 on both sides of the wall 100. The implant 24 shown on Figs. 13 and 14 is, for its own part, designed for receiving a prosthetic valve and enabling its assembly on a wall or similar corporeal zone. In this case, a portion 10a corresponding to slightly less than the longitudinal half of the structure 10 is folded on the other portion 10b of this structure 10 then is folded radially towards the outside at its portion of free end 10c, to form thus one of both collars 26. The end portion 10d of the other portion 10b of the structure 10 opposite portion 10a is folded radially towards the outside, and enables to form the other collar 26.

As previously, the structure 10 thus deformed is placed in a contention device which maintains it in this shape and is then exposed to a single or to various appropriate thermal treatments stabilizing its shape and conferring super elastic properties thereto. The implant 24 receives also a watertight sheet which covers said implant, notably made of Teflon.

As appears from the foregoing, the invention provides a method of production of a medical implant with mesh-like structure, notably of a "stent" or of a "plug", relatively easy to implement and enabling the realization of implants 10, 23, 24 remaining perfectly functional.

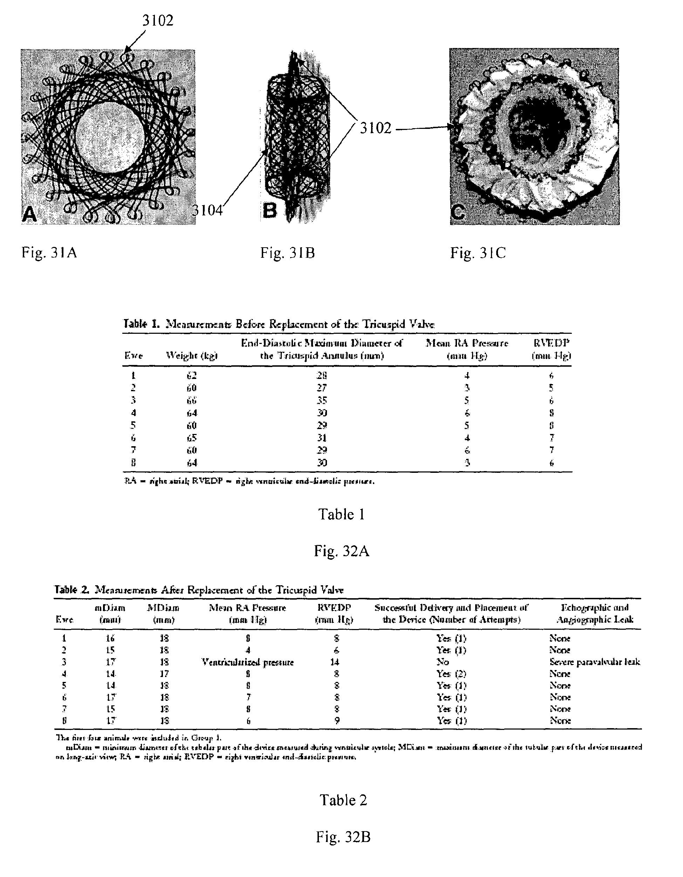

The stent illustrated in Figs. 10-12 may be particular application for use in some heart conditions such as, for example, pulmonary artery hypertension and hypoplastic left heart syndrome (see also Figs. 31A-C). Specifically, there is a need to create fluid communication between the right and the left atriums. Currently, the creation of such communication is accomplished by creating a hole using either radiofrequency, a stiff wire, a knife or a stiff needle. The hole is then dilated using a balloon catheter to increase its size. However after a period of time (days to weeks), the opening usually and spontaneously closes and needs to be redone. To avoid spontaneous closure and healing, the device according to Figs. 10-12 may be inserted into the opening to keep the opening from closing. The stent may also be used with a membrane covering (e.g., PTFE) may be self expandable. As shown, the stent may include two disks 26 separated by a tubular part 25. The two disks can have different diameters and the length of the tubular part is preferably fixed, though the stent can be provided with different lengths from zero to any length (1, 2, 3,..., -MM, -CM) depending on the thickness of the cardiac wall where the device is inserted.

The same device shown in Figs. 10-12, and especially the device shown in Figs. 13-14, may also be used for closure of a cardiac defect (hole) between chambers of the heart, though with the tubular (center) section being plugged (e.g., with a membrane or screw/plug). In such embodiments, the center tubular portion may include a very small diameter such that a small screw/plug could be used (e.g., in the case of using the stent illustrated in Figs. 10-12).

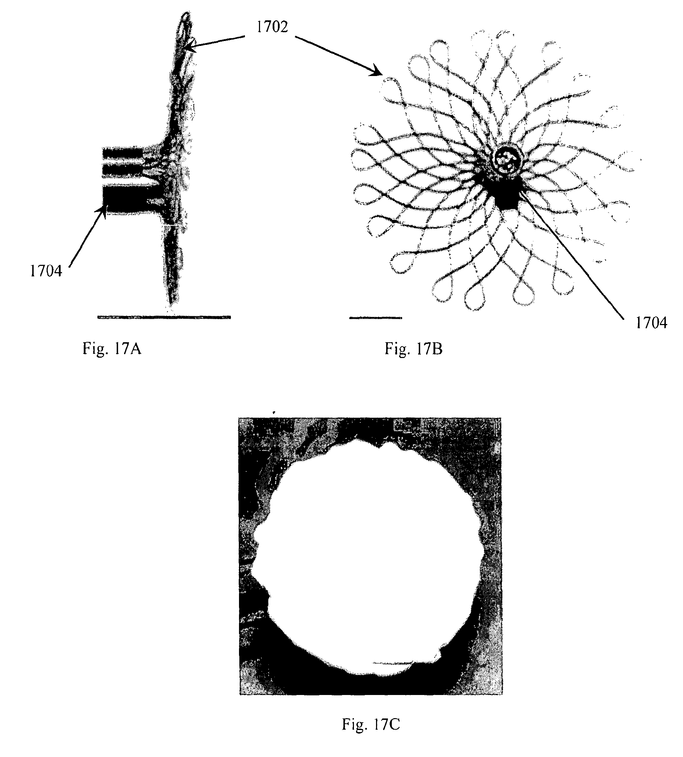

Figs. 17A-C illustrate lateral and top views, respectively, of another stent for a cardiac closure application. As shown, a first disk 1702 having a plurality of wires which are truncated and wrapped at portion 1704. The wires/structure may be wrapped using a collar or one of the strands of wire itself; or any other device which confines the bunched wires. Such a stent is preferably covered with a membrane (e.g., PTFE) as shown in Fig. 17C.

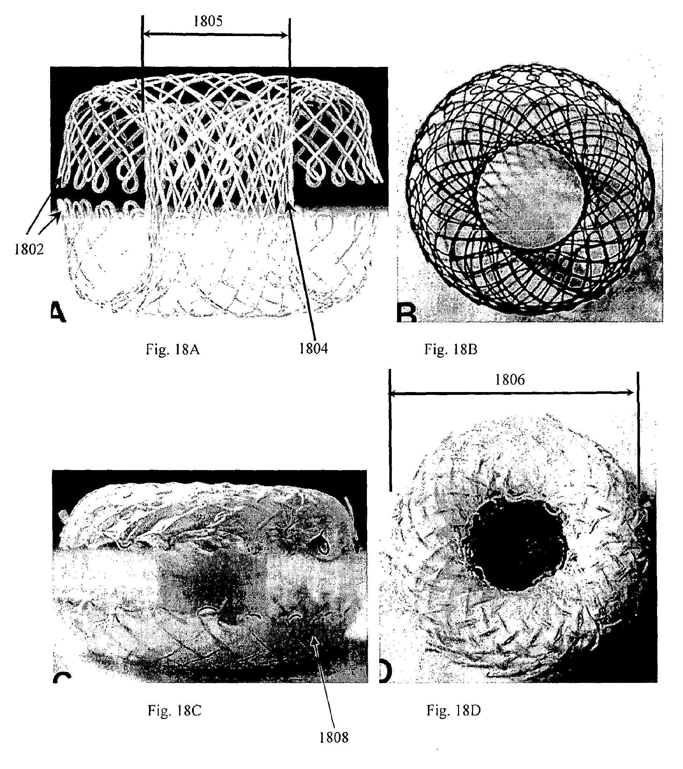

The above noted methods of manufacture of stents may be used in making other embodiments of the invention as set out below. Accordingly, Figs. 18A-D illustrate different views of a diametrical reducing, self-expanding stent according to one embodiment of the present invention. Figs. 18A-B illustrates a front view and side view, respectively, of the stent, preferably made of a single length of wire (preferably, a memory shaped alloy) of, for example, 0.22 mm (e.g., nitinol wire). Figs. 18C-D represent corresponding views of Figs. 18A-B, respectively, of the same stent but including a PTFE membrane 1808 to assure sealing of the device when used.

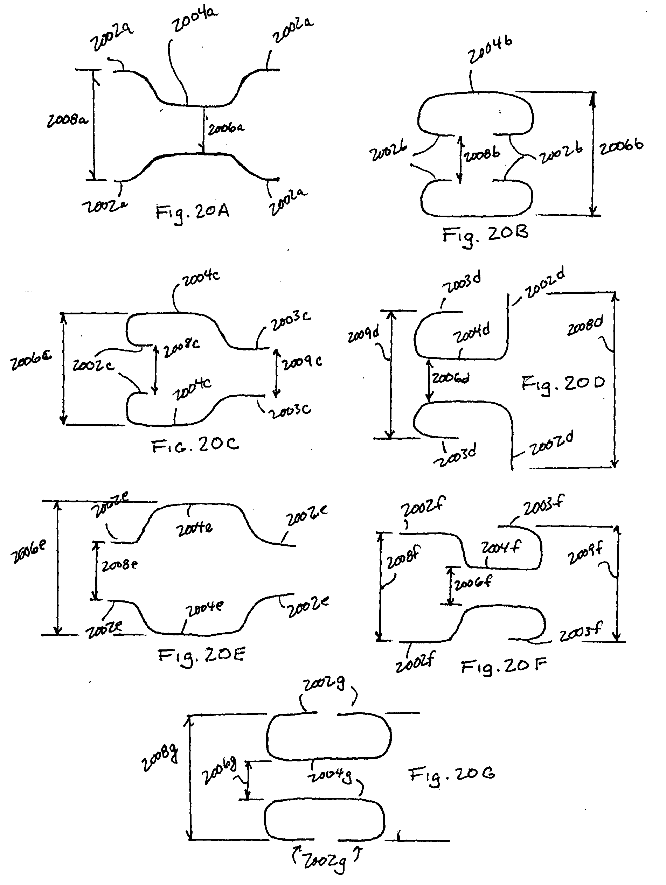

As shown in Figs. 18A-D, the ends 1802 of the diametrical reducer stent may be directed back toward the middle of the reduced diameter portion (wall structure 1804). The ends may be parallel to the walls of the reduced diameter portion, as shown in Figs. 18A-D, or the ends 1902 may simply be directed back and outwardly as shown in Figs. 19. A valve (e.g., a cardiac replacement valve) may be placed within the internal diameter, i.e., wall structure 1804. An example is shown in Fig. 19, which shows valve 1905.

To that end, one of skill in the art will appreciate that the ends of the diametrical reducer stent may be manufactured in a number of different shapes, where the reduced diameter portion and ends may be configured differently. Figs. 20A-G illustrate, generally, cross-sectional views of end configurations of diametrical reducer stents according to some embodiments of the present invention and may include such configurations where:
- the end portions 2002a may be directed outward and external (away) from wall structure 2004a (Fig. 20A) having a diameter 2006a, thereby forming a diameter 2008a;
- the end portions 2002b are directed inward and internal (Fig. 20B) to a wall structure 2004b having a diameter 2006b, thereby forming a diameter 2008b;
- one end portion 2002c being directed inward and internal to wall structure 2004c having a diameter 2006c, thereby forming a diameter 2008c, and the other end portion 2003c being directed inward and away from wall structure 2004c, thereby forming a diameter 2009c (Fig. 20C);
- one end portion 2002d being directed outward (preferably in a substantially perpendicular manner) to wall structure 2004d having a diameter 2006d, thereby forming a diameter 2008d, and the other end portion 2003d being directed outward and back toward a middle portion of wall structure 2004d, thereby forming a diameter 2009d (Fig. 20D);
- the end portions 2002e being directed inward and external to wall structure 2004e having a diameter 2006e, thereby forming a diameter 2008e (Fig. 20E);
- one end portion 2002f being directed outward and external (away) from wall structure 2004f having a diameter 2006f, thereby forming a diameter 2008f, and the other end portion being directed outward and back toward a middle portion of wall structure 2004f, thereby forming a diameter 2009f; and
- the end portions 2002g being directed outward and back toward a middle portion of wall structure 2004g having a diameter 2006g, thereby forming a diameter 2008g.

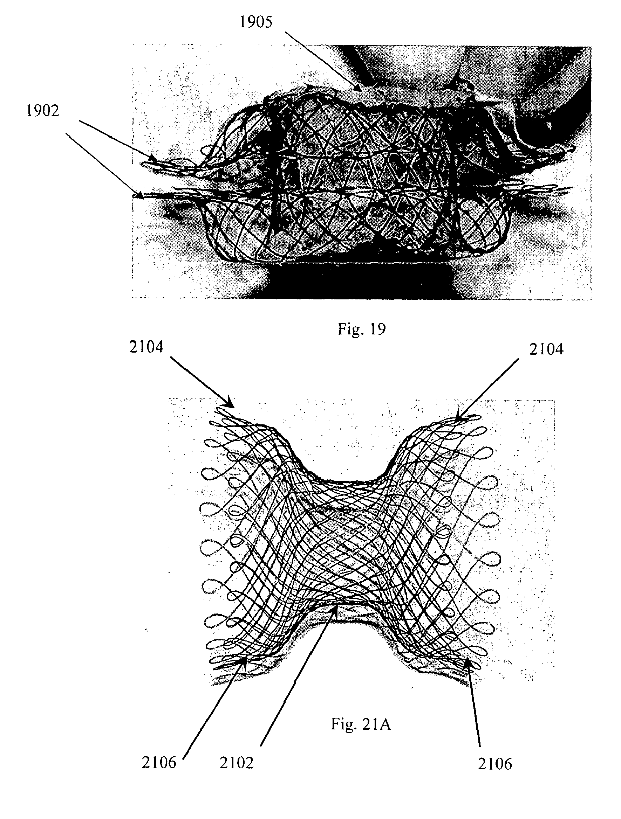

Figs. 21A-C represent examples of manufactured stents with regard to outward and external configurations. Fig. 21 A illustrates a lateral view for diametrical reducer stent having a tubular wall structure 2102 of a first diameter, where both end portions 2104 of the wall are directed outwardly and externally forming a second wall structure 2106 according to a second diameter. Preferably, at least a portion (and more preferably all) of the structure is covered with a membrane (e.g., PTFE).

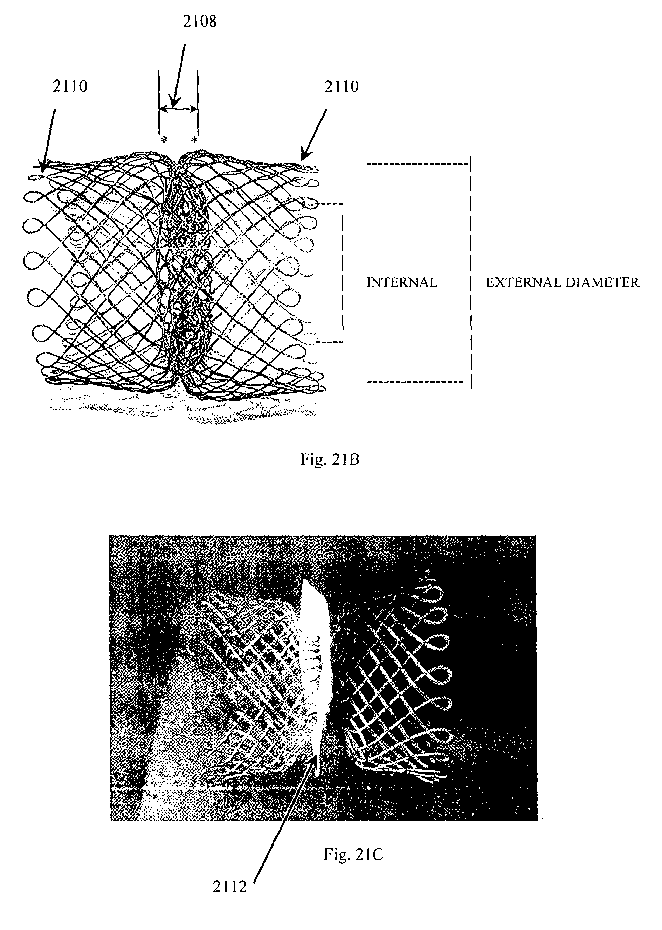

Fig. 21B illustrates a lateral view of another stent structure according to the outward and external configurations. In this design, the length of a tubular wall structure of a first diameter 2108 is reduced to a minimum (preferably), and both end portions 2110 of the wall are directed outwardly and externally forming a second wall structure according to a second diameter. In one aspect of this embodiment, at least a portion (and more preferably all) of the structure is covered with a membrane (e.g., PTFE). In another aspect of this embodiment, PTFE membrane 2112 is "sandwiched" in between end sections. An example of this is shown in Fig. 21C.

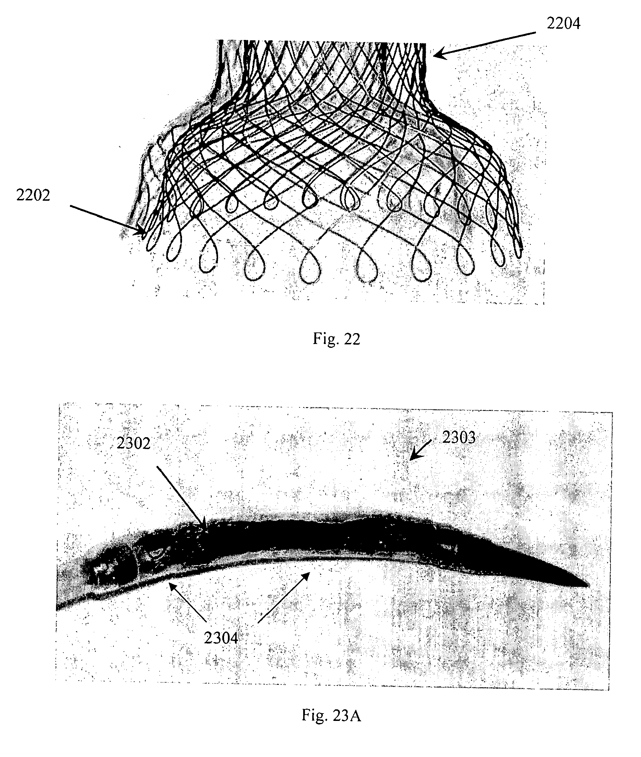

Fig. 22 illustrates another embodiment according to the present invention, which is a stent comprising an inferior portion 2202, which may be used to affix the stent to a tissue wall, and a superior, reduced diameter portion 2204. Advantageously a balloon expandable stent can be fixed to the superior part making this part dilatable to any diameter (e.g., with the use of a balloon of the proper diameter). Since the superior portion 2204 is not affixed, it is possible to reduce the diameter of this portion by using a crimper positioned between the wall vessel and the external part of the balloon expandable stent, enabling two-way reduction.

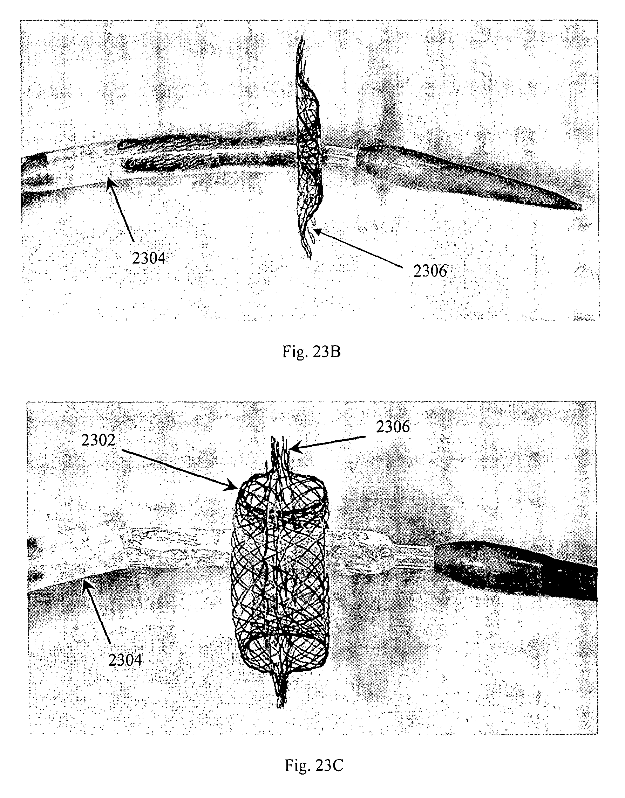

The stent of Figs. 18A-D, as well as other stents according to embodiments of the present invention, may be deployed using a sheath-type system (e.g., Cook Inc., Charenton le Pont, France). In a collapsed state, stents according to some embodiments of the present invention are considerably narrower than their corresponding expanded shapes. As shown in Fig. 23A, a stent 2302 according to an embodiment of the invention in a collapsed state is contained in a sheath deliver device 2304. It is worth noting that in a collapsed state, a stent often has a longer length than in the deployed state (e.g., sometimes over twice as long). Fig. 23B illustrates a distal portion 2306 of the stent being deployed from the sheath 2304, and a full deployment of the stent 2302 from the sheath 2304 in Fig. 23C.

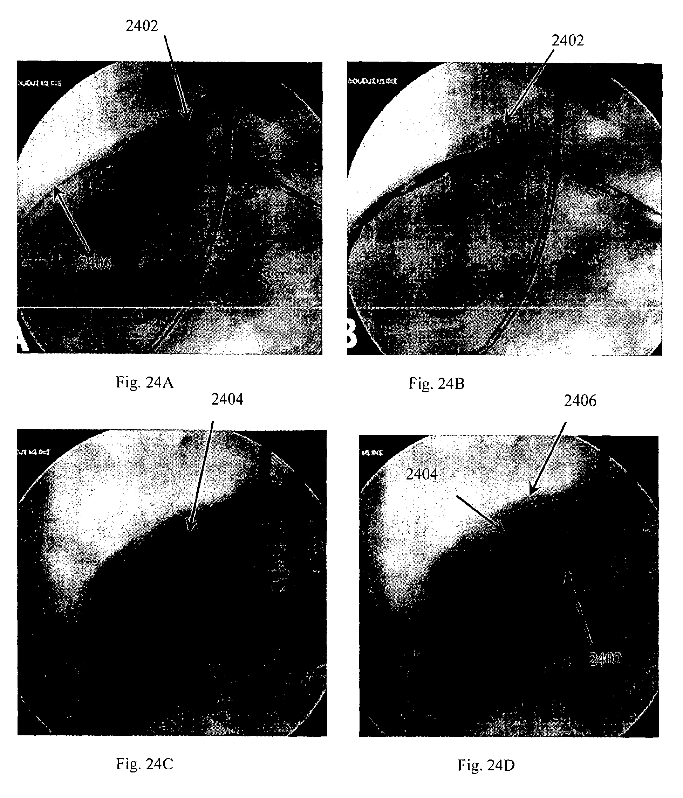

One particular advantage of some embodiments of the present invention is the implanting of stents, and in particular, deployment of diametrical reduction stents. Accordingly, X-ray images of the deployment of a stent according to some embodiments of the present invention are shown in Figs. 24A-D, which illustrate deployment of a stent from a sheath deployment device/system. Accordingly, Figs. 24A-B illustrate the deployment of a distal portion 2402 of the stent, followed by deployment of the proximal part 2404. Fig. 24D illustrates a final aspect of the stent 2406. In one example, the stent is loaded into the delivery system 2303, inserted over a previously inserted and positioned guide-wire 2405 leading to the area of interest (e.g., pulmonary artery). The stent is then advanced into the area of interest. The distal portion 2402 may be deployed (Fig. 24A) by pulling on the external sheath (see Fig. 23B, external sheath 2304, distal portion 2306) while maintaining the rest of the delivery system in position; the dital end then emerges. Upon the distal end being deployed, it is then orientated in a correct manner by pushing on the delivery system. This enables the ends of outer diameter to be turned back (i.e., inverted) toward a center of the sent, so that the memory alloy is configured to its predetermined shape (Fig. 24B). The tubular portion of the sent may then be subsequently uncovered (Fig. 24C), followed by the proximal portion of the diametrical reducer stent (Fig. 24C) yielding a final configuration as shown in Fig. 24D. After the complete delivery of the stent, the delivery system may be retrieved leaving the device in position.

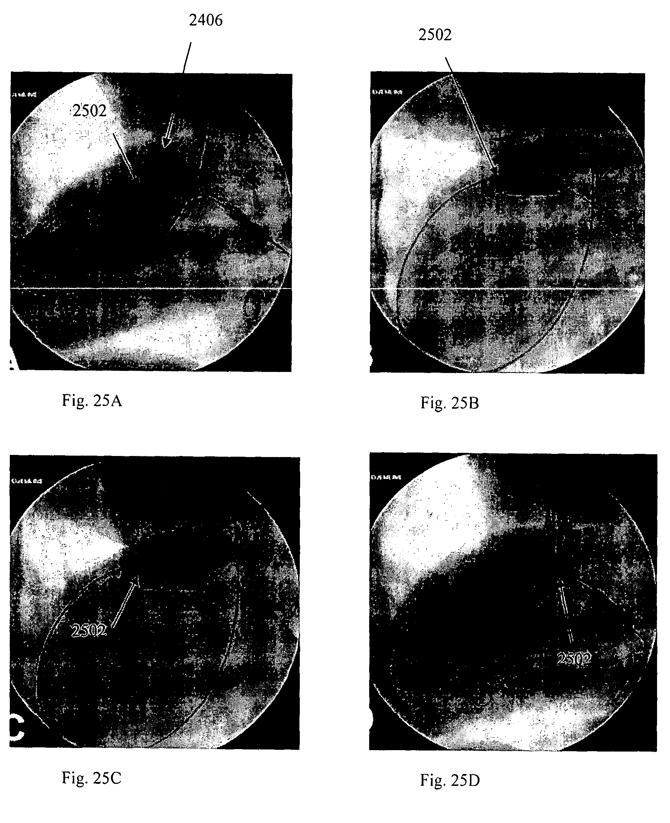

Figs. 25A-D are X-ray images of the placement of a valved (for example) stent system according to one embodiment of the present invention, which includes two separate stents, either of which may be self-expanding or balloon expandable. While a self-expanding valved stent may be used in such an application, the figures illustrate the use of a balloon expandable stent. Accordingly, Fig. 25A is an angiogram showing the insertion of the valved stent 2502 inside the first diametrical reducer stent 2504 (see Figs. 18A-D). Fig. 25B illustrates expansion of an inner balloon catheter to expand the stent 2502, and Fig. 25C illustrates expansion of an outer balloon catheter. Fig. 25D illustrates the final, full deployment of the two-stent system.

The stent system, as shown in Figs. 25A-D, where a balloon expandable stent having, for example, a valve, in the tubular, reduced diameter portion of a diametrical reducer stent includes the following advantages. First, it allows a stepwise delivery of a valve replacement system, where a diametrical reducer stent is first gradually deployed (distal portion then proximal portion), and then deployment of the valved stent, to be placed in the reduced diameter portion of the reducer stent, preferably after complete configuration of the self-expanding diametrical reducer stent. Second, such a multi-component system may allow one to caliber the tubular, reduced diameter portion of the diametrical reducer stent to a desired diameter, and may also allow for the adjustment of the diameter of the tubular portion (e.g., increasing the diameter over a period of time). In fact, this two stent system may be used without a valve to calibrate the tubular portion of the diametrical reducer stent.

While some embodiments of the present invention present a multi-component, valved (or unvalved) stent system, other embodiments of the present invention may include diametrical reducer stents have a valve component sutured (or otherwise attached) into the tubular, reduced diameter portion of the stent. Such an embodiment is illustrated in Fig. 19.

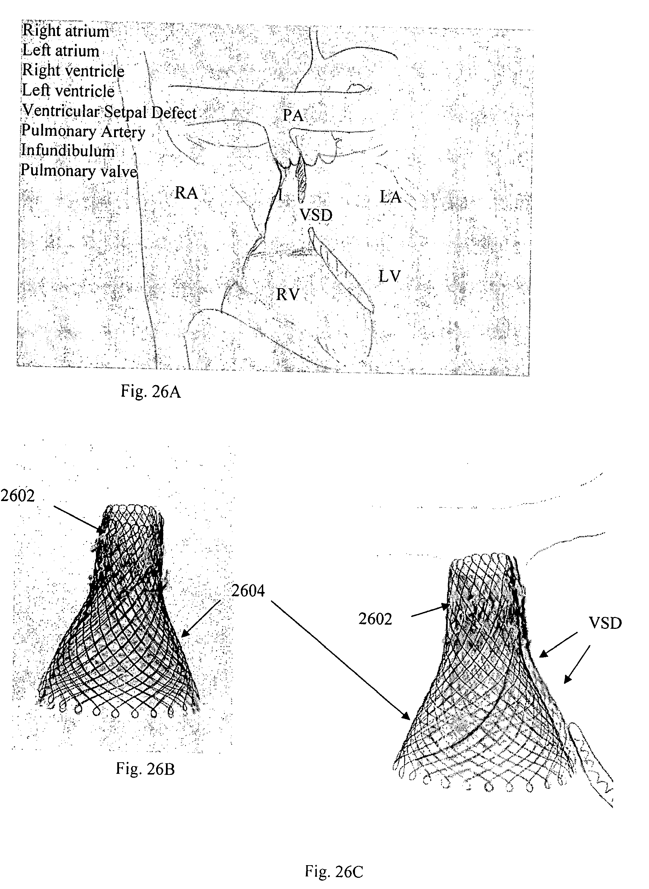

Figs. 26A-C illustrate yet another embodiment of the present invention, which may be particular advantageous for use in the treatment of a staged, right-ventricular outflow tract stenosis (e.g., subvalvular, valvular and supravalvular) and ventricular septal defect closure. Fig. 26A is cross-sectional diagram illustrating a heart with the congenital heart defect - a tetralogy of Fallot, associating a ventricular setpal defect (VSD), a sub=-pulmonary stenosis, a pulmonary valve stenosis. This defect is usually treated by surgery.

As shown in Fig. 26B, the stent, which may be self-expanding, includes an upper tubular portion 2602 and a lower conical portion 2604. The device is preferably covered with a PTFE membrane allowing for simultaneous closure of the ventricular septal defect (Fig. 26A) as shown in Fig. 26C (the PTFE is not represented in the figure). A balloon expandable stent may be added on or in the tubular portion thereby enabling a staged delivery and increasing the radial strength for complete opening of the device and the stenosis; the tubular portion has released the supra-valvular, the valvular stenosis and the infundibulum obstruction. The balloon expandable stent may additionally have a valve included in the upper portion (for example) to avoid the loss of valvular function when the device is inserted.

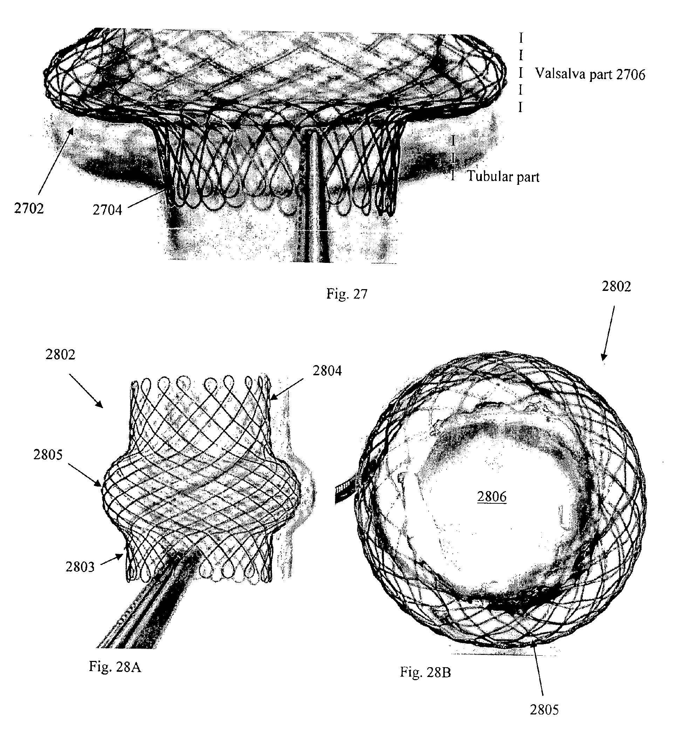

Fig. 27 illustrates a stent which may be used, for example, in pulmonary and/or aortic valve replacement, through a mini-invasive procedure (surgery or transcatheter for example). Such a stent includes a bulged portion corresponding to the Valsalva sinus region of a valve (e.g., aortic valve). The tubular portion 2604 of the stent 2704 according to the present embodiment corresponds to an area that retains a valve, and a bulged portion 2706 which mimicks the Valsalva sinus of the patient. Such an arrangement includes the following advantages. First, the structure of present embodiment mimicks the natural geometry of the aortic with a potential of hemodynamic improvement. Moreover, the stent need not be orientated since it may be inserted below the coronary orifices. Furthermore, the stent may enlarge the present indication to patients with aortic regurgitation and large aortic annulus.

This stent also preferably includes a covering (e.g., PTFE), covering at least the tubular portion and the portion of the Valsalva section (not shown). Fig. 27 illustrates the stent without the valve inserted. To that end, the valve may be sutured directly within the tubular portion (allowing for a one step procedure without balloon requirement), or inside a balloon expandable stent that is sutured inside the tubular part. The latter embodiment includes the advantage of allowing for a two step or staged procedure: delivery and the positioning of the Valsalva portion, then positioning the valved stent and inflating the balloon to open the valved stent in the tubular portion. Another advantage of the latter embodiment is that the stent having the Valsalva portion can be recaptured up until inflation of the ballon expandable stent. Thus, if the Valsalva portion is inadequate, the device can be retrived prior to placement of the balloon expandable stent having the valve by recapturing or reloading the device inside the delivery system.

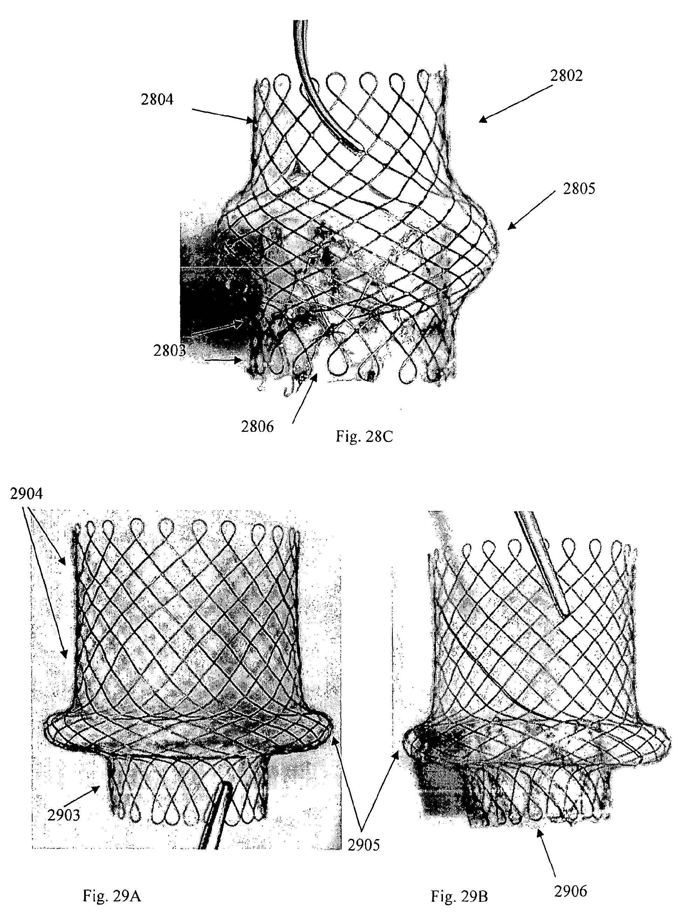

Figs. 28A-C illustrate yet another embodiment of the present invention. As show, stent 2802 includes an additional second tubular portion 2804 to increase the available fixation surface to the pulmonary wall, and larger bulge portion 2805 (as that in Fig. 27). The length and diameter of this second tubular portion may be different from the first tubular portion 2803, but may also be identical. Preferably, the second tubular portion is longer and of larger diameter than the first tubular portion. Preferably, the stent is also covered by a PTFE membrane. Figs. 28B-C illustrate the stent with a valve 2806 placed inside, the valve being in a closed position.

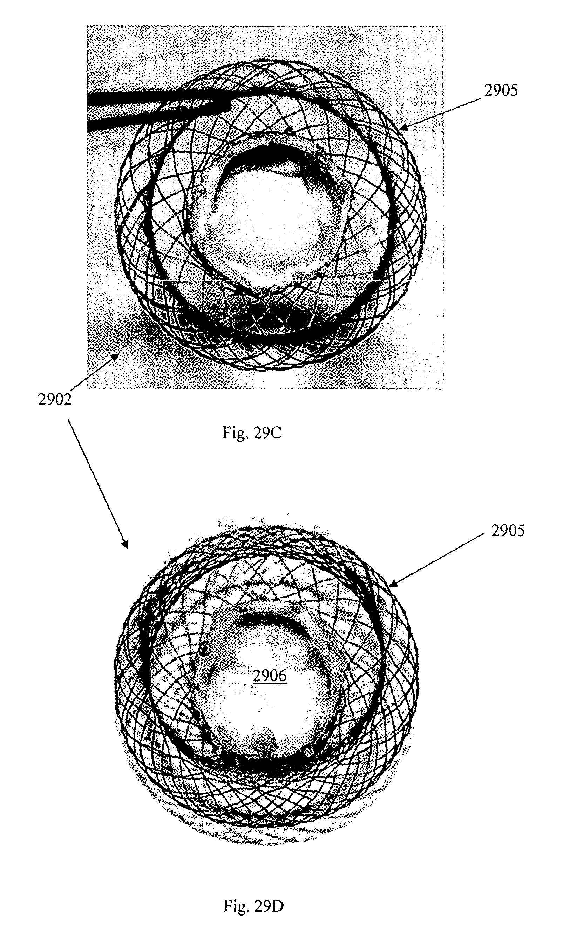

Fig. 29A-D illustrates yet another embodiment of a stent 2902, similar to the stent of Figs. 28A-C, but with a narrower bulge portion 2905, with a first tubular portion 2903, a second tubular portion 2904. Fig. 29B illustrates a front view showing a valve 2906 inside the device. Figs. 29C-D illustrate a front view of the stent, with the valve in the open (Fig. 29C) and closed (Fig. 29D) positions.

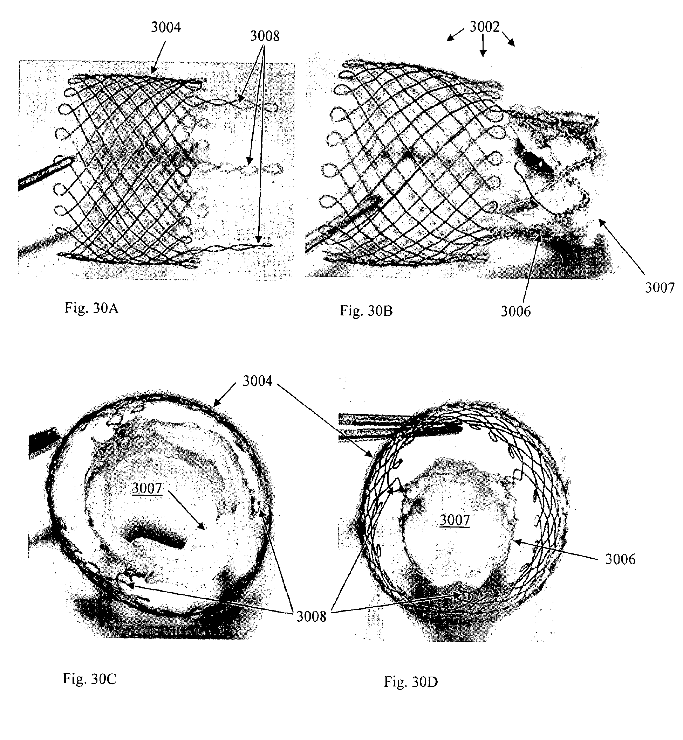

Figs. 30A-D illustrate a stent system 3002 which includes improvements for positioning the stent related to coronary orifices and mitral valve, and anchoring of the stent; Fig. 30A illustrates the subject stent without a valve, and Figs. 30B-D illustrating the stent with a valve 3007. Accordingly, the stent includes two components: a first component 3004 for assuring the fixation to the ascending aorta, and a second component 3006 for retaining the valve. The two components may be linked by tentacles or prolongations 3008 of the stent fixed in the ascending aorta. Such tentacles may be radio-opaque and may help to orientate the stent in the coronary orifices. One additional advantage of such a structure may include the ability to modify a distance between the two components during insertion, as well as the stent containing the valve can or not slide over the prolongation (as rails) making possible to modify the distance between the 2 two stents during the insertion and to configure the device regarding the anatomy of each patient.

The distance between the two interdependent components in the figures is short, but can be longer and may be made adjustable - via the prolongations noted above. Since the prologations may be fixed to the stent containing the valve at a precise level, the device is capable of being orientated. The larger diameter component 3004 acts as a holder and may be fixed to the wall of the ascending aorta, while the other stent component 3006 holds the valve 3007 to the annulus. Note that the prolongations 3008 can alternatively originate from the stent component 3006 containing the valve. One stent may be made of self expandable materiel and the other one (with the valve) is balloon expandable. This, as set out in other embodiments of the invention, allows a stepwise delivery with first positioning of stent 3004 to the ascending aorta and once the orientation is correct, the expansion of the balloons and of the valved stent component 3006. Alternatively, the 2 stents can be self-expandable or balloon expandable.

Example - Percutaneous Replacement of Atrioventricular Valves

Device description. A self-expandable symmetrical stent constructed from a 0.22-mm nitinol wire was designed. The overall length when deployed was 15 mm. It is formed by two flat disks 3102 and a tubular portion 3104. The disks and the central part had a spontaneous diameter of 40 mm and 18 mm, respectively (see Fig. 31A). It is braided using a single wire, making all parts physically interconnected. When deployed, the disks tended to join in the middle of the tubular part (Fig. 31B). Because of this design and the alloy properties, the tendency of apposition of these disks created forces that fixed the device around the annulus with one disk being deployed in the right ventricle (RV) and one in the right atrium (RA). Additionally, the tubular part interconnected between the disks acted as a supporting structure for the valve to be implanted.

Device preparation. A naturally valved venous segment, harvested from the bovine jugular vein (Contegra, Medtronic Inc., Minneapolis, Minnesota) was prepared and mounted into the tubular part of the self-expandable stent. To guarantee the sealing of the device, we sutured a polytetrafluoroethylene (PTFE) membrane, usually used for covered stents (Zeus Inc., Orangeburg, South Carolina), on the outside of the ventricular disk (Fig. 31 C). The atrial disk was not covered to limit the risk of coronary sinus occlusion. All devices were stored in glutaraldehyde solution until their use.

The delivery system. The delivery system consisted of a "homemade" front-loading 18-F long sheath (Cook Inc., Charenton le Pont, France). For the purpose of the study, the distal tip of a dilator was cut off. A piece of catheter was fixed to this part to liberate space for stent placement. The length of this space (i.e., piece of catheter) was 5.5 cm, which was the length of the device when in the constrained position. At the tip of the catheter, a 1-cm-long dilator was attached to allow for a smooth transition between the tip and the sheath and to facilitate the tracking of the delivery system during its course. The sheath could freely slide over the device. No balloon was necessary to deliver the nitinol stent that spontaneously deployed at the time of uncovering.

Preparation of the animals. Animals were treated according to the European regulations (9), and the protocol was approved by the institutional ethics committee. Eight ewes weighing 60 to 70 kg were included. We intended to implant in the tricuspid position a device sheltering an 18-mm valve as a one-step procedure. Animals were divided into two equal groups according to the killing time points. General anesthesia was induced with 10 mg/kg of thiopental and maintained with isoflurane. Right jugular and femoral veins were prepared for catheterization.

Percutaneous replacement of the tricuspid valve. Through the right jugular vein, a 5-F right Judkins coronary catheter (Cordis, Issy les Moulineaux, France) was advanced in the distal right pulmonary artery (PA). Through this catheter, a 0.035-inch extra-stiff guidewire (Amplatzer, Golden Valley, Minnesota) was positioned distally. The valved device was loaded into the delivery system, inserted over the previously positioned wire, and advanced into the RV. As with devices for closure of atrial septal defects, the distal disk was deployed in the RV by pulling on the external sheath while maintaining the dilator in position. This disk was then applied to the tricuspid annulus by pulling on the external sheath and dilator. After deployment of the tubular part containing the valve, the second disk was delivered similarly in the RA (see also Figs. 24A-D). The two disks sandwiched the annulus, with one disk laying into the RV and the proximal one in the RA.

Epicardial echocardiography imaging and cardiac catheterization. The RV and RA pressures were measured before and after device implantation. Angiographic evaluation consisted of an atrial injection and a right ventriculography. A small left thoracotomy was performed in all animals to allow for an epicardial echocardiography. Angiograms and echocardiography were initially performed to define the anatomy of the area of interest and to measure the maximum diameter of the tricuspid annulus. Studies were also repeated after implantation and before killing to confirm the appropriate position and sealing of the device and to verify the function of the implanted valves. In animals with tricuspid regurgitation, a RV angiography was performed through the RV because the regurgitation could be enhanced or created by the position of the catheter through the implanted valve.

Graft retrieval. Grafts were electively explanted one hour (group 1) and one month (group 2) after valve implantation. The subvalvular area was examined to determine the relationship between the cordae and the device and the position of the implanted device in relation to the tricuspid valve annulus. After cutting down the interventricular septum, the device was harvested with the RA and RV free wall and rinsed to remove excess intraluminal blood. Valvular competency was grossly tested by passing fluid in the graft. The RA was finally dissected and inspected macroscopically to look for injuries and for the position of the proximal disk.

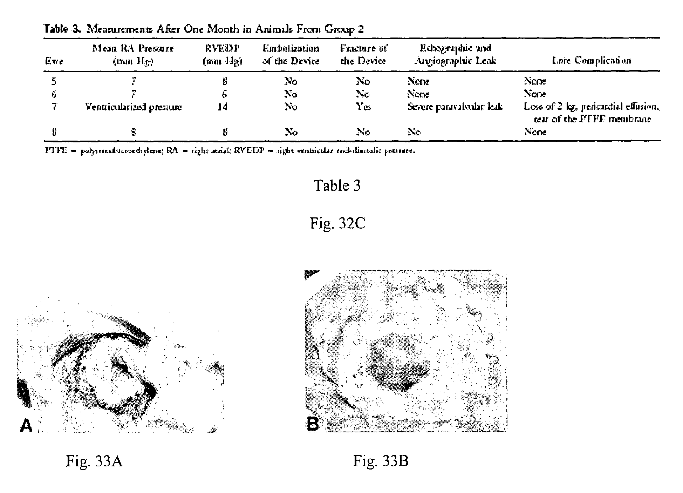

RESULTS. The mean maximum diameter of the tricuspid annulus was 30 mm, ranging from 27 to 35 mm. The mean RA pressure increased from 5 to 7 mm Hg after valve implantation (range 4 to 8 mm Hg) (see Figs. 32A-B, Tables 1 and 2). Unsustained ventricular and atrial ectopic beats occurred during wire placement and device deployment in all animals. No sustained or hemodynamically relevant arrhythmias were recorded during the study. Short-termevaluation (group1). In group 1, three of four devices were successfully implanted with good function of valves (Fig. 32B, Table 2). In one animal, it was impossible to completely deploy the valve despite attempts to dilate the device with a balloon catheter. In this animal, the device was not aligned with the tricuspid annulus. Systemic blood pressure subsequently decreased, and the QRS enlarged with ST-segment depression. Angiographic and echocardiographic evaluations showed a severe paravalvular leak. At autopsy, the ventricular disk was trapped in the tricuspid cordae, explaining its incomplete deployment. In one animal, the dilator glued on the tip of the delivery system embolized in the PA. In another ewe, echographic data acquired during deployment showed that the ventricular disk was incorrectly opened in the RA. Because the device was not fully deployed, it was possible to reload it in the RA by pushing on the Mullins sheath while maintaining the dilator and the wire positions. After reloading, the delivery system was re-advanced in the RV and the device was delivered properly.

One-month evaluation (group2). In group 2, all devices were successfully implanted. The mean RA pressure did not significantly change when comparing acute and chronic evaluations (7 vs. 7.3 mm Hg). There was no early or late stent migration (Fig. 32B-C, Tables 2 and 3). Evaluations showed that implants were in the desired position and confirmed the sealing of the device, showing no significant leak in three of four animals (see Figs. 24A-D). In one animal, a significant paravalvular leak was found at the one-month evaluation. At autopsy, a pericardial effusion was found and the PTFE was torn beside a weld fracture. Elsewhere, valves were competent and no other stent fractures were found. At autopsy, valve leaflets were thin and mobile in all animals. All devices were sitting in the area of the tricuspid annulus. Devices were partially covered by a fibrous tissue, making devices impossible to retrieve without structural damage (Figs. 33A-B). No macroscopic damage was noted when inspecting the right cavities. As expected, the proximal and distal disks were respectively in the RA and the RV. The tricuspid native valve was completely inactivated by the stent and partially retracted.

DISCUSSION. No data are presently available on percutaneous valve replacement of atrioventricular valves. Before the availability of mitral homograft valves, semi-lunar valves were used for that indication. For percutaneous implantation the use of such valves is possible, but several difficulties must be resolved. First, the discrepancy between the size of the available transcatheter valve and the size of the annulus makes the use of current stent designs impossible. Second, the valve must be anchored to the annulus not to embolize. Third, in the case of percutaneous reduction of the annulus size, the device must ensure the perfect sealing of the gap between the true and reduced annulus diameters. Therefore, it was necessary to develop a device for this indication that fulfilled the previous criteria. For that purpose, we designed a new self-expandable stent formed of two disks separated by a tubular part. The diameter of the two disks was chosen to be slightly larger than the diameter of the tricuspid annulus to allow for anchoring. Mechanical fixation was ensured by trapping the annulus between the two disks. An 18-mm valve was sutured in the tubular part of the device.

Finally, the PTFE covering guaranteed the sealing of the careful echographic assessment before complete release of device. The implantation of these newly designed stents was the device. Although it has not seemed to be necessary to feasible in seven of eight ewes. The implantation of this time the delivery of the device in coordination with a newly designed device permitted the reduction of the specific phase of the cardiac cycle, it could be important to annulus diameter to the desired diameter with no significant avoid entrapment in the tricuspid cordae. Techniques of increase in RA pressure. This hemodynamic finding did not rapid ventricular pacing or vagal stimulation probably change in any animals with "late" killing time points. We need to be investigated further. No migration occurred failed to implant one valved device because it was trapped in early or during the follow-up. A significant paravalvular the tricuspid cordae. This should be avoided by a more leak occurred in one animal. At autopsy at one-month follow-up, the PTFE membrane was torn beside a weld fracture.

Having now described a few embodiments of the invention, it should be apparent to those skilled in the art that the foregoing is merely illustrative and not limiting, and it should be understood that numerous changes in size, shape and configuration of the disclosed embodiments may be introduced without departing from the true spirit of the invention as defined in the appended claims.

## Claims

1. A medical implant comprising a stent having a tubular first wall structure (1804, 2004g) of a first diameter (1805, 2006g), wherein at least one end portion (1802, 2002g) of the first wall structure is formed into a second diameter (1806, 2008g) and wherein the combination of the first wall structure (1804, 2004g) and the second diameter (1806, 2008g) form a diametrical reducer.

2. The medical implant according to claim 1, wherein the stent is self-expanding.

3. The medical implant according to claim 1, wherein the at least one end portion of the first wall structure is directed either:
outwardly from the first wall structure at substantially a perpendicular angle;
outwardly and back from the first wall structure toward an intermediate position of the stent forming a second wall structure according to a second diameter;
inwardly and internal to the first wall structure;
inwardly and away from the first wall structure;
outwardly from the first wall structure to form a conical portion; or
outwardly and away from the first wall structure.

4. The medical implant according to claim 1, further comprising a membrane (1808) covering at least a portion of the implant.

5. The medical implant according to claim 1, further comprising a valve (1905) positioned within the implant or within the first wall structure.

6. The medical implant according to claim 1, wherein the implant comprises an interlaced, helicoidally wound wire forming a mesh-like structure.

7. A medical implant system comprising:
a first self-expanding stent (2406) comprising a tubular first wall structure of a first diameter, wherein at least one end portion of the first wall structure is formed into a second diameter; and
a second balloon expandable stent (2502) comprising a tubular structure, wherein the balloon expandable stent is deployed within a portion of the first wall structure of the first stent.

8. The implant system according to claim 7, further comprising a valve positioned within the first wall structure of the first self-expanding stent, or within the tubular structure of the second balloon expandable stent.

9. A self-expanding valve replacement implant (2802) for a patient comprising a first tubular wall structure (2804) of a first diameter and a Valsalva portion (2805) corresponding in shape to the Valsalva portion of the patient.

10. The self-expanding implant according to claim 9, further comprising a valve (2806) positioned in the first tubular wall structure.

11. The self-expanding implant according to claim 9, further comprising a second tubular wall structure (2803) having a second diameter larger that the first diameter.

12. The self-expanding implant according to claim 11, wherein the Valsalva portion is provided between the first tubular wall structure and the second tubular wall structure.

13. A medical implant comprising a first stent (3004) having a first tubular wall structure of a first diameter and a second separate stent (3006) comprising a second tubular wall structure of a second diameter, wherein at least one of the first stent and the second stent includes at least one prolongation (3008) which connects the first stent and the second stent in spaced apart arrangement.

14. The medical implant according to claim 13, wherein a distance between the first stent and the second stent is adjustable.

15. The medical implant according to claim 13, wherein at least one of the first stent and the second stent is self-expanding.

16. The medical implant according to claim 13, wherein at least one of the first stent and the second stent is balloon expandable.

17. The medical implant according to claim 13, wherein a valve (3007) is included with either the first stent or the second stent.

18. A system for implanting a medical device comprising:
a self-expanding stent (2302) capable upon expansion of forming a tubular first wall structure of a first diameter, wherein each end portion of the first wall structure form into a second wall structure of a second diameter larger than the first diameter;
a delivery system (2303) having a sheath capable of loading the self-expanding stent; and
a guide-wire (2405) upon which the delivery system is guided to an area of interest in a patient for implanting the stent, wherein upon the delivery system being advanced to the area of interest, the distal end of the stent is deployed from the sheath, the delivery system is pushed in the distal end direction such that distal end of the stent is turned backward toward the proximal end of the stent.

19. The system for implanting a medical device according to claim 18, wherein subsequent to the distal end of the sent being turned backward, the tubular wall structure of the stent is deployed and then the proximal end of the stent is deployed.

20. The system according to claim 18, further comprising a second stent (2502) comprising a second tubular structure, capable of being deployable within the first wall structure of the first stent.

21. The system according to claim 18, further comprising a valve for positioning within the stent.
